(19) Europäisches Patentamt
European Patent Office
Office européen des brevets

(11) Publication number : **0 286 316 B1**

(12) **EUROPEAN PATENT SPECIFICATION**

(45) Date of publication of patent specification : **12.02.92 Bulletin 92/07**

(51) Int. Cl.[5] : **C07D 323/04,** C07D 493/04, A61K 31/335, // (C07D493/04, 323:00, 303:00)

(21) Application number : **88302879.7**

(22) Date of filing : **30.03.88**

(54) **1,2,4-Trioxane Derivatives.**

The file contains technical information submitted after the application was filed and not included in this specification

(30) Priority : **03.04.87 CH 1311/87**

(43) Date of publication of application : **12.10.88 Bulletin 88/41**

(45) Publication of the grant of the patent : **12.02.92 Bulletin 92/07**

(84) Designated Contracting States : **AT BE CH DE ES FR GB GR IT LI LU NL SE**

(56) References cited :
**JOURNAL OF THE CHEMICAL SOCIETY, CHEMICAL COMMUNICATIONS, 1984, pages 523,524; C.W. JEFFORD et al.: "Reactions of cyclic peroxides with aldehydes and ketones catalysed by trimethylsilyl trifluoromethanesulphonate. An efficient synthesis of 1,2,4-trioxanes"**

(56) References cited :
**JOURNAL OF THE AMERICAN CHEMICAL SOCIETY, vol. 105, 1983, pages 624,625; G. SCHMID et al.: "Total synthesis of qinghaosu"**
**JOURNAL OF THE AMERICAN CHEMICAL SOCIETY, vol. 105, 1983, pages 6497,6498; C.W. JEFFORD et al.: "Reaction of bicyclic endoperoxides with carbonyl compounds. A new approach to 1,2,4-trioxanes"**
**CHEMICAL ABSTRACTS, vol. 106, no. 15, 13th April 1987, page 645, column 1, abstract no. 119857g, Columbus, Ohio, US; C.W. JEFFORD et al.: "Intramolecular oxygen transfer on the ozonolysis of a diphenylcyclopentene derivatve", & HELV. CHIM. ACTA 1986, 69(4), 941-948 (Cat. A,D)**

(73) Proprietor : **Jefford, Charles W.
49 Chemin de Saussac
CH-1256 Troinex Geneva (CH)**

(72) Inventor : **Jefford, Charles W.
49 Chemin de Saussac
CH-1256 Troinex Geneva (CH)**

(74) Representative : **Ruffles, Graham Keith et al
MARKS & CLERK 57-60 Lincoln's Inn Fields
London WC2A 3LS (GB)**

**EP 0 286 316 B1**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

BACKGROUND OF THE INVENTION

This invention relates to derivatives of 1,2,4-trioxane, and to pharmaceutical uses of the derivatives.

Tropical diseases such as malaria can be treated by nitrogen-containing agents, including quinine, chloroquine, mefloquine or pyrimethanine. Parasites (Plasmodium falciparum) resistant to such medicinal substances, in particular to chloroquine, have developed, making the fight against these diseases much harder.

Recently, a new antimalarial agent which is a polyoxygenated tetracyclic molecule has been isolated from the shrub Artemisia annua L and named "Qinghaosu" (see for example Science, 228. 1049 (1985). Qinghaosu has the structure:

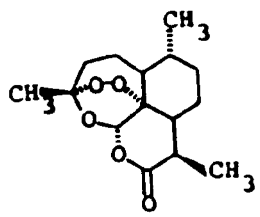

It is remarkably active against chloroquine-resistant strains of P. falciparum, but synthesis is difficult and commercially impractical. Moreover, its preparation from the α- and β- lactols affords no particular advantages. Presently, the only source of Quinghaosu is by extraction from crops of Artemisia annua L in small and variable yields.

SUMMARY OF THE INVENTION

It has now been discovered that a new class of purely synthetic compounds, in particular certain fused derivatives of 1,2,4-trioxane, exhibit interesting pharmaceutical properties, in particular anti-matarial properties.

This invention provides for the pharmaceutical use of a 1,2,4-trioxane derivative of formula:

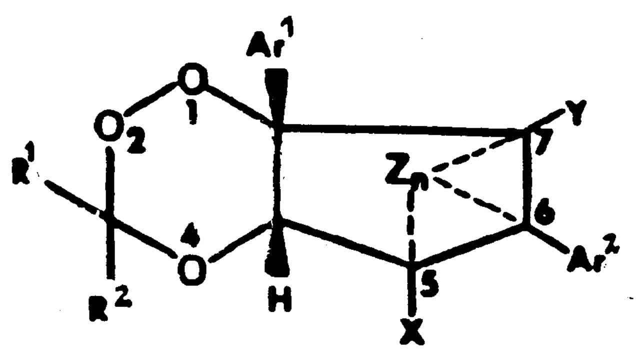

(I)

wherein

the subscript n is equal to 0 or 1;

the symbol Z represents an epoxide oxygen atom at the 5,6 or 6,7 positions, or a pair of electrons forming a double bond at the 5,6 or 6,7 positions;

each of the symbols $Ar^1$ and $Ar^2$, being the same or different, represents an aromatic group which is optionally substituted;

each of the symbols $R^1$ and $R^2$, being the same or different, represents a linear or branched alkyl group, which is optionally substituted, or $R^1$ and $R^2$, taken together with the carbon atom to which they are attached, form an alicyclic group which is optionally interrupted by one or more oxygen, sulphur or nitrogen atoms and which group is optionally substituted; and

each of X and Y, being the same or different, represents a hydrogen atom or a functional group which contains oxygen, nitrogen or sulphur.

The present invention embraces racemic mixtures, as well as one or other of the enantiomeric forms.

PREFERRED EMBODIMENTS OF THE INVENTION

The compounds of this invention are of interest because of their synthetic origin. Representative compounds are particularly active as antimalarial agents, more so than chloroquine or mefloquine. Form in vitro and in vivo tests, the present 1,2,4-trioxane derivatives exhibit equal or higher activity than Qinghaosu, in particular against chloroquine-resistant clones of P. falciparum. Some of the compounds have shown, according to appropriate tests, interesting immunosuppressive activity. Consequently, the compounds are of utility for the treatment of tropical diseases such as malaria, or for the treatment of diseases of immunological nature or viral origin.

Hence, the present invention provides pharmaceutical compositions which comprise a compound of the formula (I), together with a pharmaceutically acceptable carrier.

The compounds of the present invention have very low toxicity, permitting massive doses to be given to patients at the brink of death. For example, the compounds can be formulated as suspensions in oil and injected intramuscularly as a single shot. While formulations for parenteral administration may be used, it is within this invention to formulate the compounds as formulations for oral administration. Tablets for prophylactic treatment are especially suitable. To this end, some modifications of the compounds may be desirable, for example to enhance water solubility through inclusion of p- or m- carboxyl or amino substituents onto the aryl substituents. Suitable modifications can be achieved by routine experiment tion, including for instance (i) etherification of a 5- or 7- hydroxy compound to give an alkyloxycarbonylmethyl ether which can be de-esterified to give a 5- or 7- oxymethylcarboxylic acid; (ii) synthesis of pyridinium derivatives by treating bis-pyridylcyclopentadiene with singlet oxygen and then a ketone $R^1R^2C=O$; (iii) synthesis of analogous compounds where $Ar^1$ and/or $Ar^2$ are p- or m-diethylaminophenyl substituents, followed by acidification to give a salt; or using other substituents on $Ar^1$ and/or $Ar^2$ for solubilization. More generally, formulation of the compounds of this invention can be based on the conventional techniques available to the pharmacist.

The subscript n can be 0 or 1. When n is 1, the symbol Z represents a 5,6-epoxy group, a 6,7-epoxy group, a double bond at the 5,6 position, or a double bond at the 6,7 position. For preference, the subscript n is 1 and Z represents a double bond.

The symbols $Ar^1$ and $Ar^2$ represent the same or different aromatic groups, which may be heterocyclic aromatic groups, for instance with 5 or 6 ring atoms with 1 or 2 oxygen, sulphur or nitrogen heteroatoms. Examples of such aromatic groups include a phenyl, p-tolyl, naphthyl or pyridyl group. The aromatic groups can be substituted, in which case the substituents comprise one or more alkyl groups such as methyl, aryl groups such as phenyl; alkoxy groups such as methoxy, hydroxy groups; halogen atoms such as chlorine; or fluorine; carboxyl groups; optionally alkyl-substituted amino groups such as diethylamino groups; alkoxycarbonyl groups; or other functional groups. The carboxyl groups may be esterified.

The symbols $R^1$ and $R^2$ can be the same or different alkyl groups, especially alkyl groups of 1 to 4 carbon atoms, in particular methyl, ethyl or n-butyl, which are optionally substituted with the substitutents illustrated for the aromatic groups $Ar^1$ and $Ar^2$. Alternatively, $R^1$ and $R^2$ with the carbon atom to which they are attached can form an alicyclic group, optionally interrupted with O,S, S=O, $S(=O)_2$ or NH, and optionally substituted with the substituents illustrated for $Ar^1$ and $Ar^2$, especially a cycloalkane of 3 to 7 carbons optionally interrupted with O or S, and optionally substituted with hydroxy or methyl, in particular a cyclopentane, cyclohexane or oxolane group.

The groups X and Y are the same or different, and each represents a hydrogen atom or a functional group which contains oxygen, nitrogen or sulphur. For preference, at least one of X and Y is a hydrogen atom. The functional groups comprise a hydroxy group, a peroxide group, an ester (such as an optionally chiral oxycarbonyl, oxycarbonyloxy or oxyalkylcarbonyloxy ester), a carboxylic acid (such as an oxyalkylcarboxylic acid), a ketone, an imine, a hydrazone, an amino acid, a peptide residue, a glycosyl group, a phosporyl group, a diphosphenyl group, or a phosphate reside.

Preferred compounds of formula (I) include those listed in the following table:

Table: Part (i)

| | code | m | n | Z* | $R^1$ | $R^2$ |
|---|---|---|---|---|---|---|
| 1 | skdj-25 | 0 | 1 | 5,6= | $CH_3$ | $CH_3$ |
| 2 | skdj-44 | 0 | 1 | 6,7= | $CH_3$ | $CH_3$ |
| 3 | skdj-41 | 0 | 1 | 5,6= | $CH_3$ | $CH_3$ |
| 4 | aj-30 | 0 | 1 | 5,6= | $-(CH_2)_4-$ | |
| 5 | aj-31 | 0 | 1 | 5,6= | $-(CH_2)_5-$ | |
| 6 | aj-12 | 0 | 1 | 5,6= | $CH_3$ | $CH_3$ |
| 7 | aj-21 | 0 | 1 | 6,7= | $CH_3$ | $CH_3$ |
| 8 | aj-18 | 0 | 1 | 6,7= | $CH_3$ | $CH_3$ |
| 9 | aj-04 | 0 | 1 | 6,7= | $CH_3$ | $CH_3$ |
| 10 | aj-19 | 0 | 1 | 6,7= | $CH_3$ | $CH_3$ |
| 11 | aj-44 | 0 | 1 | 6,7= | $-(CH_2)_4-$ | |
| 12 | jcr-pm4 | 0 | 1 | 5,6= | $-(CH_2)_2O(CH_2)_2-$ | |
| 13 | jcr-pm5 | 0 | 1 | 6,7= | $-(CH_2)_5-$ | |
| 14 | jcr-pm3 | 0 | 1 | 5,6= | $-(CH_2)_6-$ | |
| 15 | jcr-pm8 | 0 | 1 | 6,7= | $-(CH_2)_5-$ | |
| 16 | jcr-pm7 | 0 | 1 | 6,7= | $-(CH_2)_5-$ | |
| 17 | jcr-419 | 0 | 1 | 5,6= | $-(CH_2)_2CO(CH_2)_2-$ | |
| 18 | jcr-423 | 0 | 1 | 5,6= | $-CH_3$ | $-(CH_2)_3COOEt$ |
| 19 | jcrgc11 | 0 | 1 | 5,6= | $-(CH_2)_2O(CH_2)_2O\ C(CH_2)_2-$ | |
| 20 | jcrpm-2 | 0 | 1 | 5,6= | $CH_3$ | $C_2H_5$ |

Z*: in the table, "5,6" and "6,7", refer respectively to the bond between the 5 and 6 positions, and the 6 and 7 positions. A double bond is shown "=". An epoxide is shown "O".

Table: Part (ii)

| | code | X | Y | $Ar^1$ | $Ar^2$ |
|---|---|---|---|---|---|
| 1 | skdj-25 | H | H | Ph | Ph |
| 2 | skdj-44 | OH | H | Ph | Ph |
| 3 | skdj-41 | H | H | Ph | Ph |
| 4 | aj-30 | H | H | Ph | Ph |
| 5 | aj-31 | H | H | Ph | Ph |
| 6 | aj-12 | H | H | p-ClPh | p-ClPh |
| 7 | aj-21 | OCO(o-F)Ph | H | Ph | Ph |
| 8 | aj-18 | $O-CO-O-CH(CH_3)_2$ | H | Ph | Ph |
| 9 | aj-04 | =O | H | Ph | Ph |
| 10 | aj-19 | $OCOCH_2CH(CH_3)_2$ | H | Ph | Ph |
| 11 | aj-44 | OH | H | Ph | Ph |
| 12 | jcr-pm4 | H | H | Ph | Ph |
| 13 | jcr-pm5 | OOH | H | Ph | Ph |
| 14 | jcr-pm3 | H | H | Ph | Ph |
| 15 | jcr-pm8 | =O | H | Ph | Ph |
| 16 | jcr-pm7 | OH | H | Ph | Ph |
| 17 | jcr-419 | H | H | Ph | Ph |
| 18 | jcr-423 | H | H | Ph | Ph |
| 19 | jcrgc11 | H | H | Ph | Ph |
| 20 | jcrpm-2 | H | H | Ph | Ph |

Especially preferred compounds are numbers 12, 16 and close analogues thereof.

The compounds 1, 2, and 4 listed above are known from the scientific literature, without mention of any pharmaceutical properties (see on this subject Helv Chim Acta 69, 941 (1986) and J Chem Soc Chem Comm 523 (1984)).

Most of the compounds of formula (I) are new and can be synthesized by analogy with their previously described homologues, with the aid of conventional techniques.

The new derivatives of 1,2,4-trioxane may be represented by means of the following formula:

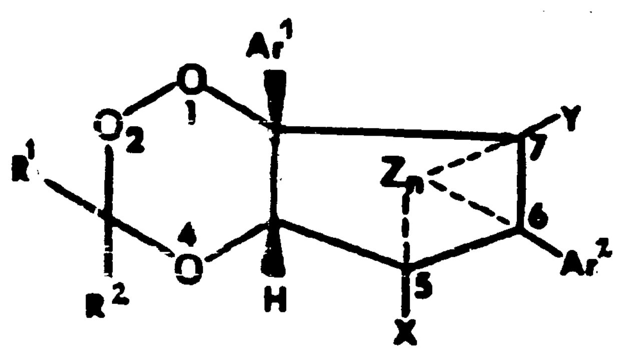

(I')

in which n, $Ar^1$, $Ar^2$, $R^1$, $R^2$, X, Y and Z are as defined for the formula (I), with the exception of the following compounds:

(a) n = 1; Z = 5,6=; $R^1 = R^2 = -CH_3$ or $R^1 + R^2 = -(CH_2)_4-$; X = Y = H; $Ar^1 = Ar^2 = Ph$.

(b) n = 1; Z = 5,6=; $R^1 = R^2 = -CH_3$; X = -OOH or -OH; $Ar^1 = Ar^2$ = Ph.

The present invention provides a process for preparing the compounds of formula (I), which comprises reaction of a ketone $R^1R^2$=O with a compound of formula:

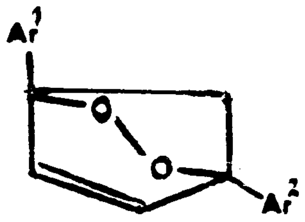

(II)

to give a compound of formula (III):

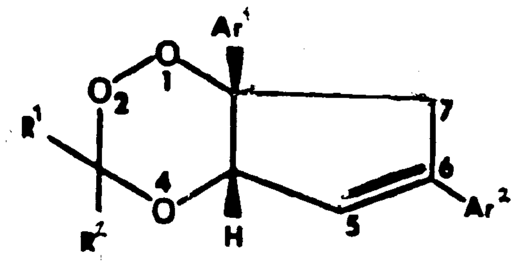

(III)

followed by derivatization as necessary.

Derivatization can follow the methods given in the literature, involving for example epoxidation of the olefin, or conversion of the 5,6-olefin, (III) with singlet oxygen to a 5-peroxy-6,7-olefin, thence hydro borohydride reduction to a 5-hydroxy-6,7-olefin, and oxidation to a 5-keto-6,7-olefin. The 5-keto-6,7-olefin can be reacted with an amine $R^aNH_2$ to give an imine, with a hydrazine $NH_2NR^bR^c$ to give a hydrazone, or catalytically reduced to remove the keto group giving the 6,7-olefin, which can thereafter be reduced to remove the olefinic unsaturation. The 6,7-olefin can itself be derivatized to give isomers of the derivatives mentioned above in the derivatization of the 5,6-olefin,.

If desired, the ketone $R^1R^2C$=O can be alicyclic and/or a pure optical isomer. The use of chiral ketones such as (-)camphor, carvone, pulegone or menthone enables stereospecific synthesis of a chiral product.

The 5,6-olefin (III) can also be oxidized at the allylic methylene group to give the corresponding $\alpha,\beta$-unsaturated ketone and thence the alcohol by reduction. The aforementioned hydroxy groups can be converted to useful esters.

EXAMPLES OF THE INVENTION

The present invention is illustrated by the following Examples for synthesis of new compounds, and in vitro test data for the anti-malarial activity of the new and known compounds as well as their immuno-modulatory activity. On the basis of the given information, a practitioner in the field will be able to develop the most appropriate derivatives and select the doses needed for the in vivo treatment of the diseases in question.

Example 1

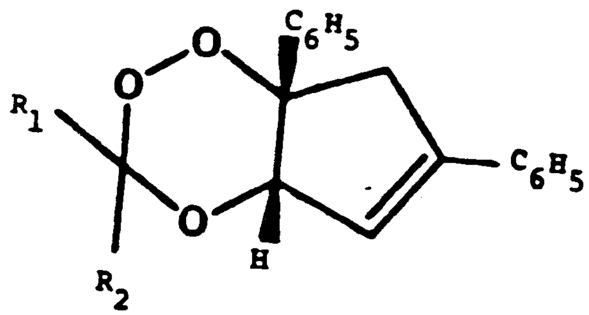

(AJ-31)

$R_1 + R_2 = (CH_2)_5$

This compound was prepared from 1,4-diphenyl-1,4-epidioxycyclopent-2-ene and cyclohexanone, according to the method published in J Chem Soc Chem Comm 523, (1984), in the presence of trimethylsilyl trifluoromethanesulfonate as catalyst in $CH_2Cl_2$ at around -78°C. The reaction mixture was treated with triethylamine and washed with water to give the product which was finally purified by recrystallization from a mixture of cyclohexane/dichloromethane, giving yellow crystals mp 102-103°C in a yield of 84%.

NMR (360 MHz, $CDCl_3$) : $\delta$ = 1.3-1.8 (8H); 1.87 (1H); 2.13 (1H); 2.98 (1H, d); 3.30 (1H, d); 5.16 (1H); 6.37 (1H, d); 7.34 (6H); 7.50 (2H, d); 7.61 (2H, d).

MS (m/e) = 348 (0,2; $M^+$), 233 (4), 218 (20), 105 (100), 91 (10), 77 (25), 55 (12).

Example 2

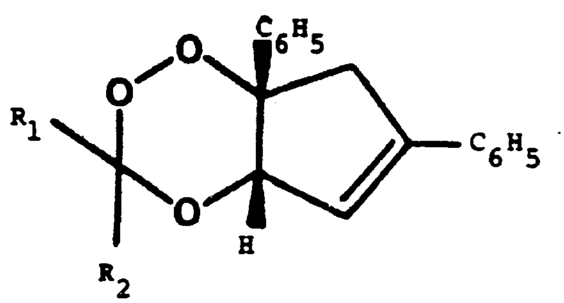

(JCR PM-4)

$R_1 + R_2 = (CH_2)_2 - O - (CH_2)_2$

This compound was prepared according to the method defined in Example 1. The cyclohexanone was replaced by an equivalent portion of 4-oxacyclohexanone. After addition of triethylamine to the reaction mixture, chromatography on silica gel and recrystallization from acetonitrile, colourless crystals were obtained, mp 145-60°C, in a yield of 59%.

NMR (360 MHz, $CDCl_3$) : $\delta$ = 1.75 (2H); 2.09 (1H); 2.30 (1H); 3.05 (d, 1H); 3.31 (d, 1H); 3.65-3.86 (4H); 5.24 (1H); 6.35 (1H); 7.28-7.45 (6H); 7.48 (2H); 7.59 (2H).

MS (m/e) = 350 (0,4; $M^+$), 218 (17), 105 (100), 77 (30).

Example 3

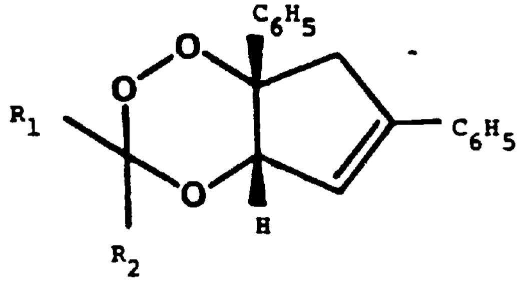

(JCR-423)

$R_1 = CH_3$; $R_2 = (CH_2)_3 - COOC_2H_5$

This compound was prepared according to the method defined in Example 1. The cyclohexanone was replaced by an equivalent portion of ethyl 5-oxohexanoate. After purification by chromatography silica gel and recrystallization, a yellow oil was obtained in a yield of 34% (mixture of 2 epimers)

NMR (200 MHz, $CDCl_3$): $\delta$ = 1.22 (3H); 1.30 (3H); 1.59 (3H); 1.65-2.10 (4H); 2.33 (2H); 2.98 (d, 1H); 3.30 (d, 1H); 4.08 (2H); 4.98 (1H); 5.07 (1H); 6.38 (1H); 7.28-7.59 (10H).

IR ($CCl_4$) : $\nu_{max}$ = 1740 $cm^{-1}$

Example 4

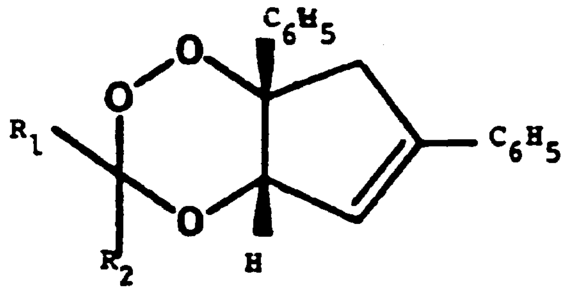

(JCR-PM-3)

$R_1 + R_2 = (CH_2)_6$

This compound was prepared according to the method defined in Example 1. The cyclohexanone was replaced by an equivalent portion of cycloheptanone, and purification by chromatography over silica gel to give colourless crystals, mp 55-60°C, yield 53%.

NMR (360 MHz, $CDCl_3$) : $\delta$ = 1.37-1.76 (9H); 1.95 (2H); 2.37 (1H); 2.99 (d, 1H); 3.33 (d, 1H); 4.92 (1H); 6.38 (1H); 7.26-7.43 (6H); 7.51 (2H); 7.56 (2H).

MS (m/e) = 250 (7), 218 (15), 105 (100), 77 (37), 68 (16), 55 (25).

Example 5

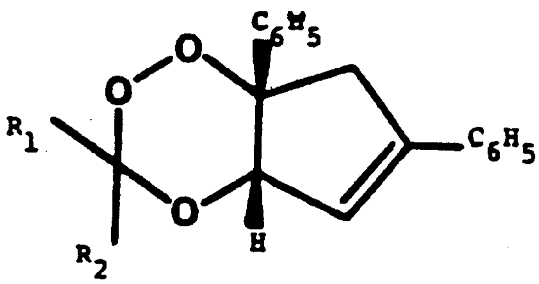

(JCR-419)

$R_1 + R_2 = (CH_2)_2 - CO - (CH_2)_2$

This compound was prepared from 1,4-diphenyl-1,4-epidioxycyclopent-2-ene and cyclohexane-1,4-dione according to the method described in Example 1 and purification by chromatography over silica gel and recrystallization from a mixture of petroleum ether/diethyl ether gave colourless crystals mp 100-101°C, yield 38%.

NMR (200 MHz, $CDCl_3$) : $\delta$ = 1.97-2.70 (8H); 3.05 (d, 1H); 3.31 (d, 1H); 5.24 (1H); 6.36 (1H); 7.28-7.55 (8H); 7.60 (2H).

IR ($CCl_4$) : $\nu_{max}$ = 1725 $cm^{-1}$

Example 6

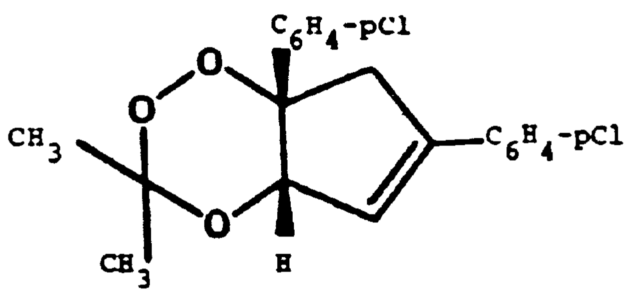

(AJ-12)

This compound was prepared according to the method described in Example 1, from acetone and 1,4-di-(p-chlorophenyl)-1,4-epidioxy- cyclopent-2-ene. The resulting crude reaction product was purified by recrystallization from a mixture of hexane/dichloromethane as colourless crystals mp 95-96°C; yield 78%.

NMR (360 MHz, $CDCl_3$) : δ = 1.24 (3H); 1.52 (3H); 2.85 (d, 1H); 3.21 (d, 1H); 4.92 (1H); 6.31 (1H); 7.35 (6H); 7. 47 (2H).

MS (m/e) = 286(5), 139(100), 111(27), 75(8).

Example 7

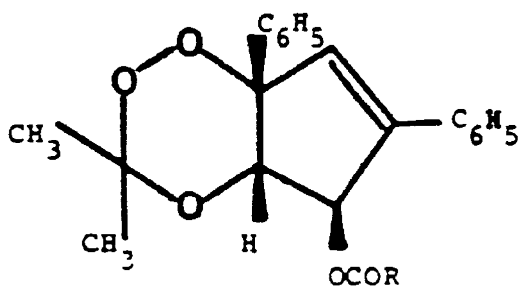

(AJ-18)

R = O-CH $(CH_3)_2$

This compound was prepared from the corresponding alcohol derivative (se Helv Chim Acta 69, 941 1986), after treatment of the latter with isopropyl chloroformate in the presence of pyridine at 0°C. After washing with water, the desired product was purified by recrystallization from hexane as colourless crystals mp 66.5-67.5°C, yield 79%.

NMR (360 MHz, $CDCl_3$) : δ = 1.21 (6H); 1.42 (3H); 1.51 (3H); 4.46 (1H); 4.91 (1H), 6.32 (m, 2H); 7.40 6H); 7.53 (2H); 7.62 (2H).

IR ($CCl_4$) : ν max = 1748 $cm^{-1}$

Example 8

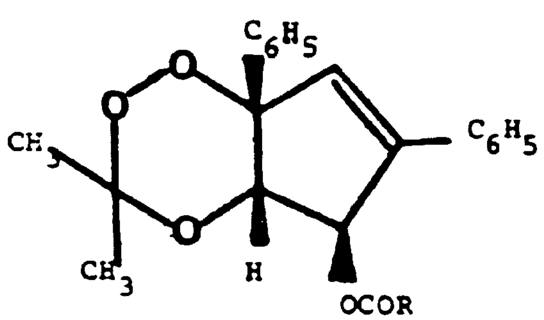

(AJ-19)

R = $CH_2$ - $CH(CH_3)_2$

This compound was prepared according to the method described in Example 7, except that isopropyl chloroformate was replaced by an equivalent portion of isovaleryl chloride. The product was purified by recrystallization from a mixture of hexane/dichloromethane as colourless crystals mp 97-80°C, yield 87%.

NMR (360 MHz, $CDCl_3$) : δ = 0.75 (6H); 1.46 (3H); 1.47 (3H); 1.88 (1H); 2.07 (2H); 4.30 (1H); 6.36 (1H); 6.39 (1H); 7.40 (6H); 7.51 (2H); 7.61 (2H).

IR ($CCl_4$) : ν max = 1743 $cm^{-1}$

Example 9

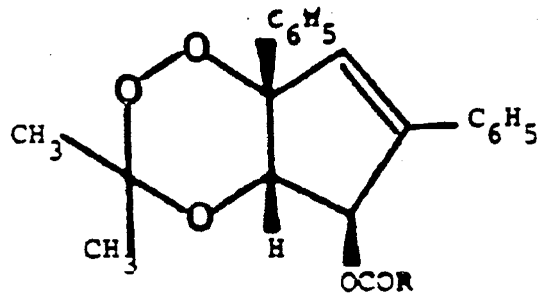

(AJ-21)

R = o-F-$C_6H_4$

This compound was prepared according to the method described in Example 7, except that isopropyl chloroformate was replaced by an equivalent portion of o-fluorobenzoyl chloride and the reaction product was purified by recrystallization from a mixture of hexane/dichloromethane as colourless crystals mp 113.5-114°C, yield 81%.

NMR (360 MHx, $CDCl_3$) : $\delta$ = 1.45 (3H); 1.50 (3H); 4.45 (1H); 6.47 (1H); 6.62 (1H); 7.09 (2H); 7.31-7.72 (12H).

IR ($CCl_4$) : $\nu_{max}$ 1723 $cm^{-1}$

Example 10

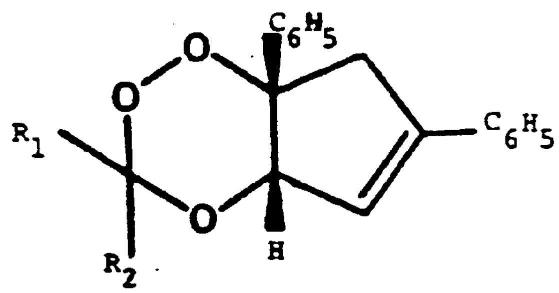

(JCR GC-11)

$R_1 + R_2 =$ 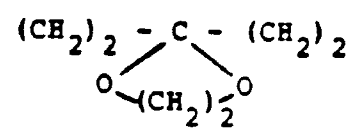

This compound was prepared from the compound of Example 5, by reaction of the latter with 1,2-di-(trimethylsiloxy)ethane, in the presence of trimethylsilyl trifluoromethanesulfonate as a catalyst (solvent $CH_2Cl_2$, temperature about -78°C). The purification was effected by chromatography over silica gel to give colourless crystals mp 126-129°C; yield 93%.

NMR: (200 MHz, $CDCl_3$) : $\delta$ = 1.40-2.50 (8H); 3.00 (d, 1H); 3.28 (d, 1H); 3.37-3.90 (4H); 5.17 (1H); 6.34 (1H); 7.22-7.65 (10H).

Example 11

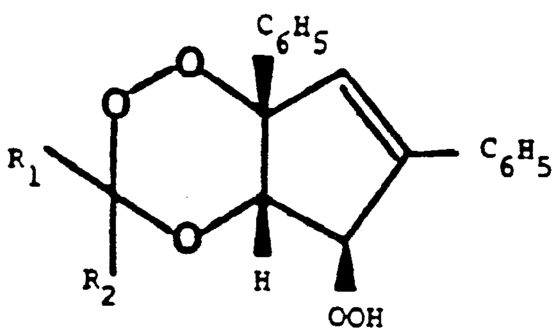

(JCR PM-5)

$R_1 + R_2 = (CH_2)_5$

This compound was prepared from the compound of Example 1, by treating the latter with singlet oxygen (see on this subject Helv Chim Acta 69, 941 (1986)) and purification by chromatography over silica gel to give colourless crystals mp 70°C, yield 90%.

NMR (200 MHz, $CDCl_3$) : $\delta$ = 1.35-2.00 (10H); 4.76 (1H); 5.59 (1H); 6.38 (1H); 7.28-7.45 (6H); 7.62 (4H); 8.05

(1H).
IR (CCl$_4$) : $\nu_{max}$ = 3545, 3460 cm$^{-1}$

Example 12

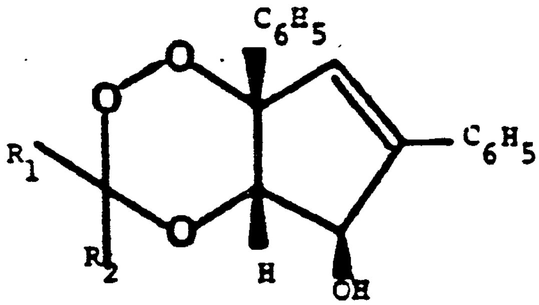

(JCR PM-7)

$R_1 + R_2 = (CH_2)_5$

This compound was prepared by reduction of the compound in Example 11 using sodium borohydride, and purification by recrystallization from a mixture of pentane/dichloromethne to give colourless crystals, yield 93%.
NMR (200 MHz, CDCl$_3$) : $\delta$ = 1.35-2.03 (11H); 4.42 (1H); 5.26 (1H); 6.30 (d, 1H); 7.30-7.48 (6H); 7.65 (4H)
IR (CCl$_4$) : $\nu_{max}$ = 3612, 3465 cm$^{-1}$

Example 13

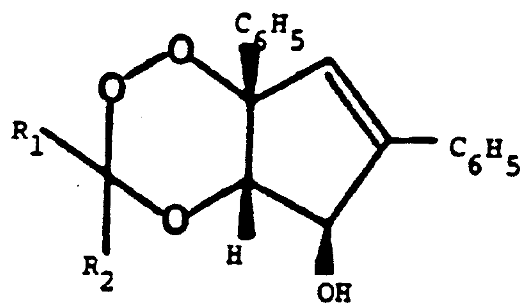

(AJ-44)

$R_1 + R_2 = (CH_2)_4$

This compound was prepared from the corresponding hydroperoxide, according to the method described in Example 12, and purification by chromatography over silica gel to give colourless crystals mp 103-104°C, yield 93%.
NMR (360 MHz, CDCl$_3$) : $\delta$ = 1.63-1.91 (6H); 2.05 (2H); 2.21 (1H); 4.43 (1H); 5.28 (1H); 6.38 (1H); 7.33-7.47 (6H); 7.62 (2H); 7.68 (2H).
IR (CCl$_4$) : $\nu_{max}$= 3620 cm$^{-1}$

Example 14

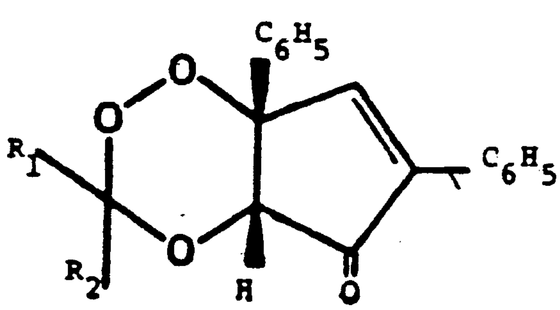

(AJ-04)

This compound was prepared from the corresponding hydroperoxide (see on this subject Helv. Chim. Acta

69, 941 (1986)), by treatment of the latter with acetic anhydride in the presence of triethylamine and purification by chromatography over silica gel followed by recrystallization from a mixture of hexane/dichloromethane to give colourless crystals mp 130-131°C, yield 83%

NMR (360 MHz, $CDCl_3$) : $\delta$ = 1.47 (3H); 1.70 (3H); 4.40 (1H); 7.47 (6H); 7.60 (2H); 7.72 (IH); 7.88 (2H).

IR ($CCl_4$) : $\nu_{max}$ = 1732 $cm^{-1}$

Example 15

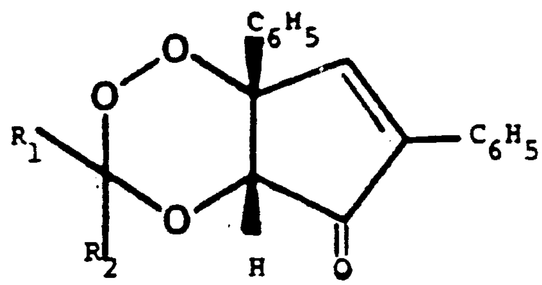

(JCR PM-8)

$R_1 + R_2 = (CH_2)_5$

This compound was prepared from the compound of Example 11, according to the method described in Example 14 giving colourless crystals, yield 30%.

NMR (200 MHz, $CDCl_3$) : $\delta$ = 1.12-2.31 (10H); 4.41 (1H); 7.48 (6H); 7.61 (2H); 7.70 (1H); 7.89 (2H).

IR ($CCl_4$): $\nu_{max}$ = 1730 $cm^{-1}$

Example 16

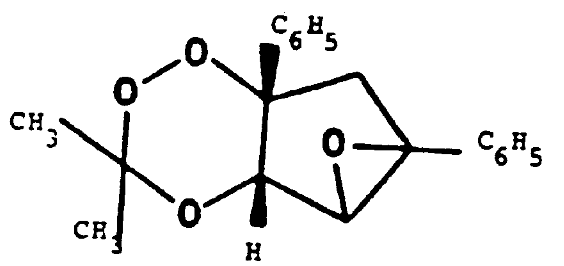

(SK DJ-41)

This compound was prepared by epoxidation of the corresponding olefin (see Helv Chim Acta 69, 941 (1986)) and purification by chromatography on silica gel, followed by recrystallization from a mixture of hexane dichloromethane giving colourless crystals of mp 88-92°C, yield 84% (2 conformers).

MS (m/e) = 147 (17), 105 (100), 77 (43).

Example 17

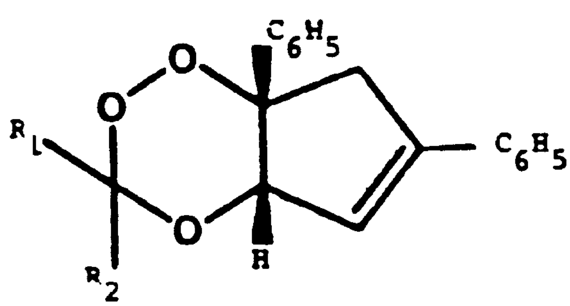

(JCR PM-2)

$R_1 = CH_3$, $R_2 = C_2H_5$

This compound was prepared from 1,4-diphenyl-1,4-epidioxycyclopent-2-ene and ethylmethyl ketone according to the procedure described in Example 1, and purified by chromatography over silica gel. A yellow

oil was obtained in 80% yield (mixture of 2 epimers according to spectral analysis).

NMR (360 MHz, $CDCl_3$): δ = 0.99 (b, 3H); 1.30 (s, 3H); 1.88 (m, 1H); 2.07 (m, 1H); 2.98 (d, 1H); 3.31 (1H); 5.01 (s, 1H); 6.37 (1H, m); 7.29-7.42 (m, 6H); 7.50 (m, 2H); 7.57 (m, 2H).

MS: m/e = 218 (36), 157 (8), 128 (11), 115 (22), 105 (100), 77 (24).

Determination of in vitro anti-malarial activities

Determination of anti-malarial activity of the compounds was effected according to the methods published by R E Desjardins et al and W K Milhous et al (Desjardins, R.E., C.J. Cranfield, D.E. Haynes, and J.D. Chulay. 1979. Quantitative assessment of anitmalarial activity in vitro by a semiautomated microdilution technique. Antimicrob. Agents Chemother. 16:710-718. Milhous, W.K., N.F. Weatherley, J.H. Bowdre, and R.E. Desjardins. 1985. In vitro activities of and mechanisms of resistance to antifol antimalarials. Antimicrob. Agents Chemother. 27:525-530). For such determinations, the following clones of Plasmodium falciparum were used:

chloroquine-resistant clone: Indochina-W2 (IndCh-W2)

chloroquine-sensitive clone: Sierra Leone-D6 (SL-D6)

The activity measured was then compared as indexes under the same conditions to the activity of Qinghaosu (I-QHS) and chloroquine (I-CHLQ), respectively. The indexes (I) provide a measure of the activity of the 1,2,4-trioxane derivatives tested in relation to the activity of the known anti-malarial substances.

The results of the determinations are summarized in the tables below.

Example 18

The known compounds shown on the next page were synthesised by the indicated literature method.

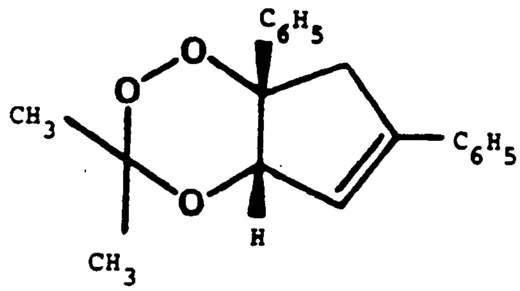

(SK DJ-25)

Helv. Chim. Acta <u>69</u>, 941 (1986)

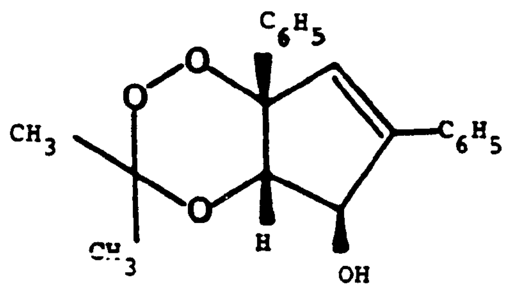

(SK DJ-44)

ditto

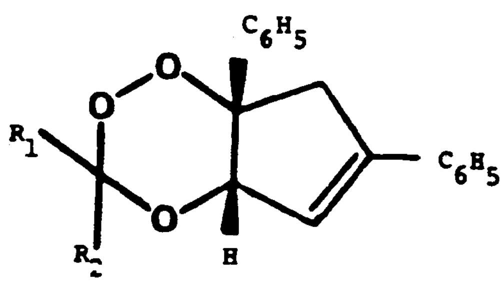

(AJ-30)

$R_1 + R_2 = (CH_2)_4$

J. Chem. Soc. Chem. Comm. <u>523</u>, 1984

Table No. 1: IndCh-W2($IC_{50}$-)

| Compound | I-QHS | I-CHLQ |
|---|---|---|
| 1 | 0.13 | 5.7 |
| 2 | 0.12 | 5.4 |
| 3 | 0.11 | 4.6 |
| 4 | 2.1 | 12.2 |
| 5 | 0.26 | 1.5 |
| 6 | 0.04 | 0.24 |
| 7 | 0.04 | 1.6 |
| 8 | 0.10 | 4.2 |
| 9 | 0.08 | 3.5 |
| 10 | 0.04 | 1.7 |
| 11 | 4.7 | 73.8 |
| 12 | 18.7 | 295.3 |
| 13 | 2.2 | 33.9 |
| 14 | 0.27 | 4.3 |
| 15 | 3.5 | - |
| 16 | 1.6 | - |
| 17 | 2.1 | - |
| 18 | 0.09 | - |
| 19 | 0.67 | - |
| 20 | 0.075 | - |

Table No. 2: S6-D6($IC_{50}$-)

| Trioxane | I-QHS | I-CHLQ |
|---|---|---|
| 1 | 0.18 | 0.20 |
| 2 | 0.25 | 0.27 |
| 3 | 0.13 | 0.14 |
| 4 | 0.79 | 0.82 |
| 5 | 0.20 | 0.20 |
| 6 | 0.06 | 0.06 |
| 7 | 0.05 | 0.06 |
| 8 | 0.16 | 0.18 |
| 9 | 0.20 | 0.21 |
| 10 | 0.09 | 0.10 |
| 11 | 1.85 | 1.16 |
| 12 | 1.10 | 0.67 |
| 13 | 1.47 | 0.93 |
| 14 | 0.13 | 0.08 |
| 15 | 1.93 | - |
| 16 | 0.49 | - |
| 17 | 1.06 | - |
| 18 | 0.28 | - |
| 19 | 0.39 | - |
| 20 | 0.36 | - |

Certain derivatives of 1,2,4-trioxane listed below were tested against mefloquine-resistant clones of Plasmodium falciparum (MEF-W2), according to the method previously cited.

Table No. 3: MEF-W2($IC_{50}$-)

| Trioxane | I-QHS |
|---|---|
| 1 | 0.15 |
| 2 | 0.10 |
| 3 | 0.05 |

Determination of immunosuppressive activity

Several of the compounds mentioned in Examples 1 to 17 were also tested for immunomodulatory activity (in vitro mitogenic assay; mixed lymphocyte reaction). The most important activity was exhibited by the compounds of Examples 2 and 6.

## Claims

1. A pharmaceutical composition which comprises a 1,2,4-trioxane derivative of formula:

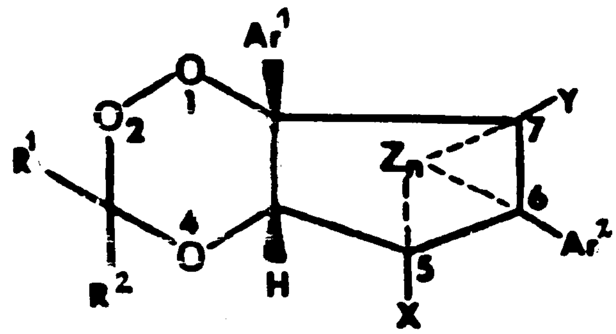

(wherein

the subscript n is equal to 0 or 1;

the symbol Z represents an epoxide oxygen atom at the 5,6 or 6,7 positions, or a pair of electrons forming a double bond at the 5,6 or 6,7 positions;

each of the symbols $Ar^1$ and $Ar^2$, being the same or different, represents an aromatic group which is optionally substituted with one or more substituents chosen from alkyl groups, aryl groups, alkoxy groups, a hydroxy group, halogen atoms, carboxy groups, optionally alkyl-substituted amino groups or alkoxycarbonyl groups;

each of the symbols $R^1$ and $R^2$, being the same or different, represents a linear or branched alkyl group, which is optionally substituted with one or more substituents chosen from alkyl groups, aryl groups, alkoxy groups, a hydroxy group, halogen atoms, carboxy groups, optionally alkyl-substituted amino groups or alkoxycarbonyl groups; or $R^1$ and $R^2$, taken together with the carbon atom to which they are attached, form an alicyclic group which is optionally interrupted by one or more oxygen, sulphur or nitrogen atoms and which group is optionally substituted with one or more substituents chosen from alkyl groups, aryl groups, alkoxy groups, a hydroxy group, halogen atoms, carboxy groups, optionally alkyl-substituted amino groups or alkoxycarbonyl groups;

each of X and Y, being the same or different, represents a hydrogen atom or a functional group which is a hydroxy group, a peroxide group, an optionally chiral oxycarbonyl, oxycarbonyloxy or oxyalkylcarbonyloxy ester, a carboxylic acid, a ketone, an imine, a hydrazone, an aminoacid, a peptide residue, a glycosyl group, a phosphoryl group, a diphosphenyl group, or a phosphate residue;

together with a pharmaceutically acceptable carrier.

2. A pharmaceutical composition according to claim 1, when formulated for parenteral administration.

3. A pharmaceutical composition according to claim 1, when formulated as a suspension in oil.

4. A pharmaceutical composition according to claim 1, when formulated for oral administration.

5. A pharmaceutical composition according to claim 1, when formulated for as a tablet.

6. A pharmaceutical composition according to any preceding claim, wherein n is 1 and Z represents a double bond.

7. A pharmaceutical composition according to any preceding, wherein at least one of X and Y is a hydrogen atom.

8. The pharmaceutical composition of claim 1, wherein said compound of formula (I) is selected from the following table (where for the values of Z, "5,6" and "6,7", refer respectively to the bond between the 5 and 6 positions, and the 6 and 7 positions; a double bond is shown "="; and an epoxide is shown "O"):

| m | n | $Z^w$ | $R^1$ | $R^2$ | X | Y | $Ar^1$ | $Ar^2$ |
|---|---|---|---|---|---|---|---|---|
| 0 | 1 | 5,6= | $CH_3$ | $CH_3$ | H | H | Ph | Ph |
| 0 | 1 | 6,7= | $CH_3$ | $CH_3$ | OH | H | Ph | Ph |
| 0 | 1 | 5,6= | $CH_3$ | $CH_3$ | H | H | Ph | Ph |
| 0 | 1 | 5,6= | $-(CH_2)_4-$ | | H | H | Ph | Ph |
| 0 | 1 | 5,6= | $-(CH_2)_5-$ | | H | H | Ph | Ph |
| 0 | 1 | 5,6= | $CH_3$ | $CH_3$ | H | H | p-ClPh | p-ClPh |
| 0 | 1 | 6,7= | $CH_3$ | $CH_3$ | OCO(o-F)Ph | H | Ph | Ph |
| 0 | 1 | 6,7= | $CH_3$ | $CH_3$ | $O-COOCH(CH_3)_2$ | H | Ph | Ph |
| 0 | 1 | 6,7= | $CH_3$ | $CH_3$ | =O | H | Ph | Ph |
| 0 | 1 | 6,7= | $CH_3$ | $CH_3$ | $OCOCH_2CH(CH_3)_2$ | H | Ph | Ph |
| 0 | 1 | 6,7= | $-(CH_2)_4-$ | | OH | H | Ph | Ph |
| 0 | 1 | 5,6= | $-(CH_2)_2O(CH_2)_2-$ | | H | H | Ph | Ph |
| 0 | 1 | 6,7= | $-(CH_2)_5-$ | | OOH | H | Ph | Ph |
| 0 | 1 | 5,6= | $-(CH_2)_6-$ | | H | H | Ph | Ph |
| 0 | 1 | 6,7= | $-(CH_2)_5-$ | | =O | H | Ph | Ph |
| 0 | 1 | 6,7= | $-(CH_2)_5-$ | | OH | H | Ph | Ph |
| 0 | 1 | 5,6= | $-(CH_2)_2CO(CH_2)_2-$ | | H | H | Ph | Ph |
| 0 | 1 | 5,6= | $-CH_3$ | $-(CH_2)_3COOEt$ | H | H | Ph | Ph |
| 0 | 1 | 5,6= | 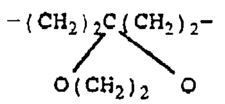 | | H | H | Ph | Ph |
| 0 | 1 | 5,6 | $CH_3$ | $C_2H_5$ | H | H | Ph | Ph |

9. A 1,2,4-trioxane of the following formula:

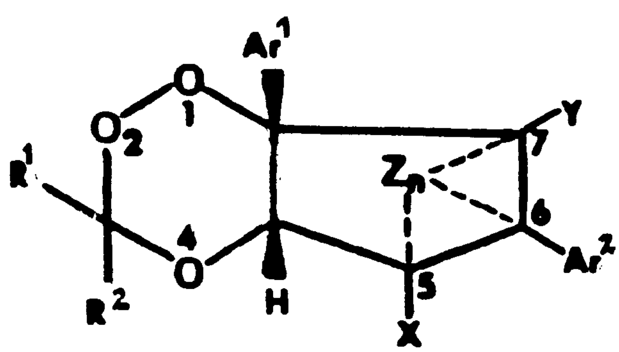

(I)

in which n, $Ar^1$, $Ar^2$, $R^1$, $R^2$, X, Y and Z are as defined in claim 1, with the exception of the following compounds:

(a) n = 1; Z = 5,6= ; $R^1 = R^2 = -CH_3$ or $R^1 + R^2 = -(CH_2)_4-$ ; X = Y = H; $Ar^1 = Ar^2$ = Ph.

(b) n = 1, Z = 6,7= ; $R^1 = R^2 = -CH_3$; X = -OOH or -OH; $Ar^1 = Ar^2$ = Ph.

10. The use of a 1,2,4-trioxane in the preparation of a pharmaceutical composition for the treatment of a human or animal body by therapy, wherein the pharmaceutical composition is in accordance with the definition given in any of claims 1 to 10.

11. The use according to claim 11, wherein the pharmaceutical composition is for the treatment of a tropical

disease such as malaria, or for the treatment of a disease of immunological nature or viral origin.

**Patentansprüche**

1. Pharmazeutische Mischung, welche ein 1,2,4-Trioxanderivat der Formel

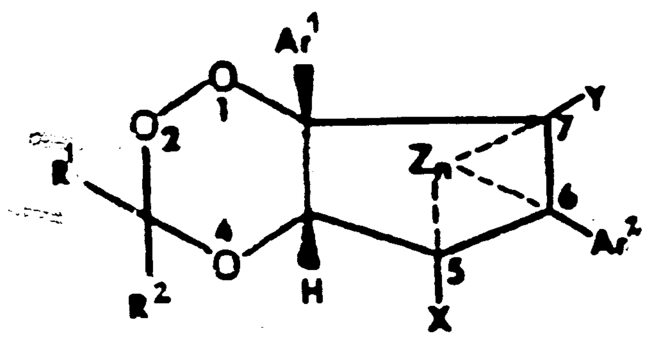

(worin

der Index n gleich ist 0 oder 1;

das Symbol Z für ein Epoxidsauerstoffatom in 5,6-Stellung oder 6,7-Stellung oder für ein eine Doppelbindung in 5,6-Stellung oder 6,7-Stellung bildendes Elektronenpaar steht,

jedes der untereinander gleichen oder voeinander verschledenen Symbole $Ar^1$ und $Ar^2$ eine aromatische Gruppe bedeutet, welche gegebenenfalls durch einen oder mehrere aus Alkylgruppen, Arylgruppen, Alkoxygruppen, eine Hydroxygruppe, Halogenatomen, Carboxygruppen, gegebenenfalls alkylsubstituierten Aminogruppen oder Alkoxycarbonylgruppen ausgewählten Substituenten substituiert ist,

jedes der untereinander gleichen oder voneinander verschiedenen Symbole $R^1$ und $R^2$ eine gegebenenfalls durch einen oder mehrere aus Alkylgruppen, Arylgruppen, Alkoxygruppen, eine Hydroxygruppe, Halogenatomen, Carboxygruppen, gegebenenfalls alkylsubstituierten Aminogruppen oder Alkoxycarbonylgruppen ausgewählten Substituenten substituierte geradkettige oder verzweigtkettige Alkylgruppe bedeutet oder $R^1$ und $R^2$ zusammen mit dem diese Reste tragenden Kohlenstoffatom eine alicyclische Gruppe bilden, welche gegebenenfalls durch ein oder mehrere Sauerstoff-, Schwefel- oder Stickstoffatome unterbrochen ist und welche gegebenenfalls durch einen oder mehrere aus Alkylgruppen, Arylgruppen, Alkoxygruppen, eine Hydroxygruppe, Halogenatomen, Carboxygruppen, gegebenenfalls alkylsubstituierten Aminogruppen oder Alkoxycarbonylgruppen ausgewählten Substituenten substituiert ist und

jeder der untereinander gleichen oder voneinander verschiedenen Reste X und Y für ein Wasserstoffatom oder eine funktionelle Gruppe steht, welche eine Hydroxygruppe, eine Peroxidgruppe, einen gegebenenfalls chiralen Oxycarbonyl-, Oxycarbonyloxy- oder Oxyalkylcarbonyloxyester, eine Carbonsäure, ein Keton, ein Imin, ein Hydrazon, eine Aminosäure, ein Peptidrest, eine Glycosylgruppe, eine Phosphorylgruppe, eine Diphosphenylgruppe oder ein Phosphatrest ist)

zusammen mit einem pharmazeutisch annehmbaren Träger enthält.

2. Pharmazeutische Mischung nach Anspruch 1, welche für parenterale Verabreichung formuliert ist.

3. Pharmazeutische Mischung nach Anspruch 1, welche als eine Suspension in Öl formuliert ist.

4. Pharmazeutische Mischung nach Anspruch 1, welche für orale Verabreichung formuliert ist.

5. Pharmazeutische Mischung nach Anspruch 1, welche als Tablette formuliert ist.

6. Pharmazeutische Mischung nach irgendeinem vorhergehenden Anspruch worin n gleich ist 1 und Z eine Doppelbindung bedeutet.

7. Pharmazeutische Mischung nach irgendeinem vorhergehenden Anspruch, worin zumindest einer der Reste X und Y ein Wasserstoffatom bedeutet.

8. Pharmazeutische Mischung nach Anspruch 1, worin die Verbindung der Formel (I) aus der folgenden Tabelle ausgewählt ist, (in welcher unter den für Z angegebenen Bedeutungen sich die Angaben "5,6" und "6,7" auf die Bindung zwischen der 5- und der 6-Stellung bzw. zwischen der 6- und der 7-Stellung beziehen, eine Doppelbindung mit "=" veranschaulicht ist und ein Epoxid als "O" dargestellt ist).

| m | n | $Z^w$ | $R^1$ | $R^2$ | X | Y | $Ar^1$ | $Ar^2$ |
|---|---|---|---|---|---|---|---|---|
| 0 | 1 | 5,6= | $CH_3$ | $CH_3$ | H | H | Ph | Ph |
| 0 | 1 | 6,7= | $CH_3$ | $CH_3$ | OH | H | Ph | Ph |
| 0 | 1 | 5,6= | $CH_3$ | $CH_3$ | H | H | Ph | Ph |
| 0 | 1 | 5,6= | $-(CH_2)_4-$ | | H | H | Ph | Ph |
| 0 | 1 | 5,6= | $-(CH_2)_5-$ | | H | H | Ph | Ph |
| 0 | 1 | 5,6= | $CH_3$ | $CH_3$ | H | H | p-ClPh | p-ClPh |
| 0 | 1 | 6,7= | $CH_3$ | $CH_3$ | OCO(o-F)Ph | H | Ph | Ph |
| 0 | 1 | 6,7= | $CH_3$ | $CH_3$ | $O-COOCH(CH_3)_2$ | H | Ph | Ph |
| 0 | 1 | 6,7= | $CH_3$ | $CH_3$ | =O | H | Ph | Ph |
| 0 | 1 | 6,7= | $CH_3$ | $CH_3$ | $OCOCH_2CH(CH_3)_2$ | H | Ph | Ph |
| 0 | 1 | 6,7= | $-(CH_2)_4-$ | | OH | H | Ph | Ph |
| 0 | 1 | 5,6= | $-(CH_2)_2O(CH_2)_2-$ | | H | H | Ph | Ph |
| 0 | 1 | 6,7= | $-(CH_2)_5-$ | | OOH | H | Ph | Ph |
| 0 | 1 | 5,6= | $-(CH_2)_6-$ | | H | H | Ph | Ph |
| 0 | 1 | 6,7= | $-(CH_2)_5-$ | | =O | H | Ph | Ph |
| 0 | 1 | 6,7= | $-(CH_2)_5-$ | | OH | H | Ph | Ph |
| 0 | 1 | 5,6= | $-(CH_2)_2CO(CH_2)_2-$ | | H | H | Ph | Ph |
| 0 | 1 | 5,6= | $-CH_3$ | $-(CH_2)_3COOEt$ | H | H | Ph | Ph |
| 0 | 1 | 5,6= | 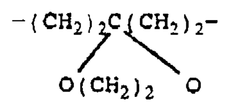 | | H | H | Ph | Ph |
| 0 | 1 | 5,6 | $CH_3$ | $C_2H_5$ | H | H | Ph | Ph |

9. Ein 1,2,4-Trioxan der folgenden Formel

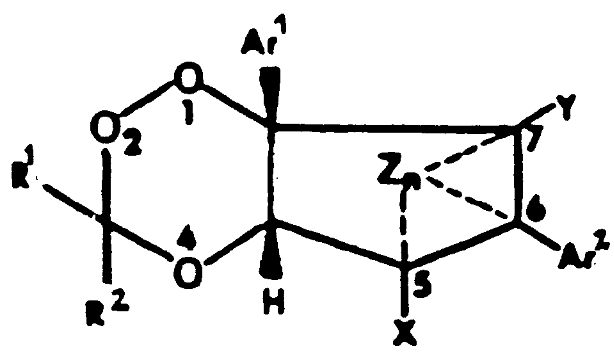

(I)

in welcher n, $Ar^1$, $Ar^2$, $R^1$, $R^2$, X, Y und Z die in Anspruch 1 angegebene Bedeutung besitzen, mit Ausnahme der folgenden Verbindungen:

(a) n = 1; Z = 5, 6= ; $R^1 = R^2 = -CH_3$ oder $R^1 + R^2 = -(CH_2)_4-$ ; X = Y = H; $Ar^1 = Ar^2$ = Ph.

(b) n = 1, Z = 6,7= ; $R^1 = R^2 = -CH_3$; X = -OOH oder -OH; $Ar^1 = Ar^2$ = Ph.

10. Verwendung eines 1,2,4-Trioxans bei der Herstellung einer pharmazeutischen Mischung zur therapeutischen Behandlung eines menschlichen oder tierischen Körpers, worin die pharmazeutische Mischung in Einklang mit der in irgendeinem der Ansprüche 1 bis 10 gegebenen Definition steht.

11. Verwendung nach Anspruch 10, worin die pharmazeutische Mischung für die Behandlung einer Tropenkrankheit wie Malaria oder für die Behandlung einer Erkrankung immunologischer Natur oder viralen Ursprungs dient.

## Revendications

1. Une composition pharmaceutique qui comprend un dérivé de 1,2,4-trioxane de formule:

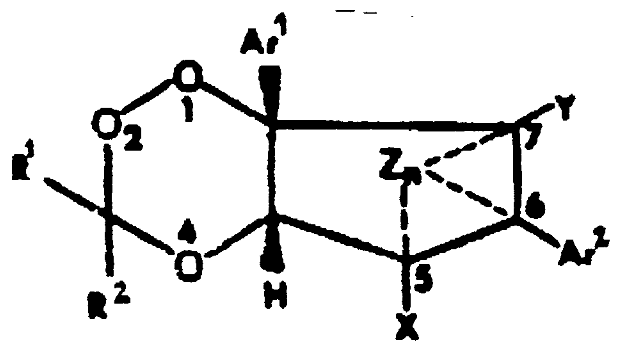

dans laquelle

l'indice n est égal à 0 ou à 1;

le symbole Z représente l'atome d'oxygène d'un groupement époxyde à la position 5,6 ou 6,7 ou une paire d'électrons formant une double liaison à la position 5,6 ou 6,7;

chacun des symboles $Ar^1$ et $Ar^2$, lesquels sont identiques ou différents, représente un groupement aromatique qui est facultativement substitué par un ou plusieurs substituants choisis parmi les groupements alkyles, les groupements aryles, les groupements alcoxy, le groupement hydroxy, les atomes d'halogènes, les groupements carboxy, les groupements aminés facultativement alkylés ou les groupements alcoxycarbonyles;

chacun des symboles $R^1$ et $R^2$, lesquels sont identiques ou différents, représente un groupement alkyle à chaîne droite ou ramifiée qui est facultativement substitué par un ou plusieurs substituants choisis parmi les groupements alkyles, les groupements aryles, les groupements alcoxy, le groupement hydroxy, les atomes d'halogènes, les groupements carboxy, les groupements aminés facultativement alkylés ou les groupements alcoxycarbonyles ou $R^1$ et $R^2$ considérés ensemble avec l'atome de carbone auquel ils sont attachés forment un groupement alicyclique qui est facultativement interrompu par un ou plusieurs atomes d'oxygène, de soufre ou d'azote, lequel groupement est facultativement susbtitué par un ou plusieurs substituants choisis parmi les groupements alkyles, les groupements aryles, les groupements alcoxy, le groupement hydroxy, les atomes d'halogènes, les groupements carboxy, les groupements aminés facultativement alkylés ou les groupements alcoxycarbonyles;

chacun des X et Y, lesquels sont identiques ou différents, représente un atome d'hydrogène ou un groupement fonctionnel, lequel est un groupement hydroxy, un groupement peroxyde, un ester oxycarbonylique, oxycarbonyloxy ou oxyalkylcarbonyloxy facultativement chiral, un acide carboxylique, une cétone, une imine, une hydrazone, un acide aminé, un résidu peptidique, un groupement glycosylé, un groupement phosphorylé, un groupement diphosphénylé ou un résidu phosphaté;

ensemble avec un véhicule pharmaceutiquement acceptable.

2. Une composition pharmaceutique suivant la revendication 1, lorsqu'elle est formulée pour une administration parentérale.

3. Une composition pharmaceutique suivant la revendication 1, lorsqu'elle est formulée sous la forme d'une suspension dans une huile.

4. Une composition pharmaceutique suivant la revendication 1, lorsqu'elle est formulée pour une administration orale.

5. Une composition pharmaceutique suivant la revendication 1, lorsqu'elle est formulée sous la forme d'un comprimé.

6. Une composition pharmaceutique suivant l'une quelconque des revendications précédentes dans laquelle n est 1 et Z représente une liaison double.

7. Une composition pharmaceutique suivant l'une quelconque des revendications précédentes, dans laquelle au moins un des X et Y est un atome d'hydrogène.

8. La composition pharmaceutique de la revendication 1, dans laquelle ledit composé de formule (1) est choisi dans le tableau suivant (où, pour les valeurs de Z, "5,6" et "6,7", on se réfère respectivement à la liaison entre les positions 5 et 6 et entre les positions 6 et 7; une double liaison est indiquée "=" et un époxyde est indiqué "O"):

| m | n | $Z^w$ | $R^1$ | $R^2$ | X | Y | $Ar^1$ | $Ar^2$ |
|---|---|---|---|---|---|---|---|---|
| 0 | 1 | 5,6= | $CH_3$ | $CH_3$ | H | H | Ph | Ph |
| 0 | 1 | 6,7= | $CH_3$ | $CH_3$ | OH | H | Ph | Ph |
| 0 | 1 | 5,6= | $CH_3$ | $CH_3$ | H | H | Ph | Ph |
| 0 | 1 | 5,6= | $-(CH_2)_4-$ | | H | H | Ph | Ph |
| 0 | 1 | 5,6= | $-(CH_2)_5-$ | | H | H | Ph | Ph |
| 0 | 1 | 5,6= | $CH_3$ | $CH_3$ | H | H | p-ClPh | p-ClPh |
| 0 | 1 | 6,7= | $CH_3$ | $CH_3$ | OCO(o-F)Ph | H | Ph | Ph |
| 0 | 1 | 6,7= | $CH_3$ | $CH_3$ | $O-COOCH(CH_3)_2$ | H | Ph | Ph |
| 0 | 1 | 6,7= | $CH_3$ | $CH_3$ | =O | H | Ph | Ph |
| 0 | 1 | 6,7= | $CH_3$ | $CH_3$ | $OCOCH_2CH(CH_3)_2$ | H | Ph | Ph |
| 0 | 1 | 6,7= | $-(CH_2)_4-$ | | OH | H | Ph | Ph |
| 0 | 1 | 5,6= | $-(CH_2)_2O(CH_2)_2-$ | | H | H | Ph | Ph |
| 0 | 1 | 6,7= | $-(CH_2)_5-$ | | OOH | H | Ph | Ph |
| 0 | 1 | 5,6= | $-(CH_2)_6-$ | | H | H | Ph | Ph |
| 0 | 1 | 6,7= | $-(CH_2)_5-$ | | =O | H | Ph | Ph |
| 0 | 1 | 6,7= | $-(CH_2)_5-$ | | OH | H | Ph | Ph |
| 0 | 1 | 5,6= | $-(CH_2)_2CO(CH_2)_2-$ | | H | H | Ph | Ph |
| 0 | 1 | 5,6= | $-CH_3$ | $-(CH_2)_3COOEt$ | H | H | Ph | Ph |
| 0 | 1 | 5,6= | $-(CH_2)_2C(CH_2)_2-$ $O(CH_2)_2$ O | | H | H | Ph | Ph |
| 0 | 1 | 5,6 | $CH_3$ | $C_2H_5$ | H | H | Ph | Ph |

9. Un 1,2,4-trioxane de la formule suivante:

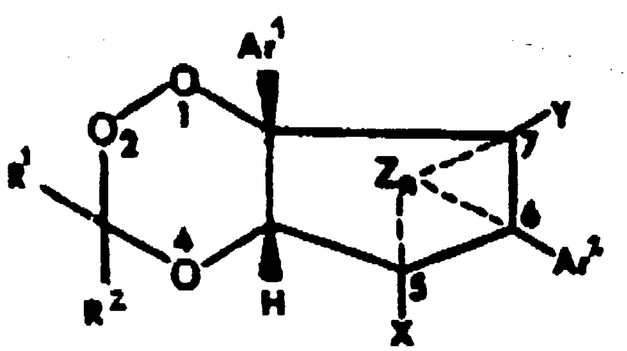

(I)

dans laquelle n, $Ar^1$, $Ar^2$, $R^1$, $R^2$, X, Y et Z sont tels que définis dans la revendication 1, à l'exception des composés suivants:

(a) n = 1; Z = 5,6 =; $R^1 = R^2 = -CH_3$ ou $R^1 + R^2 = -(CH_2)_4-$; X = Y = H; $Ar^1 = Ar^2 = Ph$.
(b) n = 1; Z = 6,7 =; $R^1 = R^2 = -CH_3$; X = - OOH ou - OH; $Ar^1 = Ar^2 = Ph$.

10. L'utilisation d'un 1,2,4-trioxane dans la préparation d'une composition pharmaceutique pour le traitement thérapeutique d'un corps humain ou animal, dans laquelle la composition pharmaceutique est en conformité avec la définition donnée dans l'une quelconque des revendications 1 à 10.

11. L'utilisation suivant la revendication 10, dans laquelle la composition pharmaceutique est destinée au traitement d'une maladie tropicale comme la malaria ou au traitement d'une maladie de nature immunologique ou d'origine virale.